# EUROPÄISCHE PATENTANMELDUNG

(11) **EP 3 085 870 A2**
(43) Veröffentlichungstag der Anmeldung: **26.10.2016**
(21) Anmeldenummer: 16166448.7
(22) Anmeldetag: 21.04.2016
(51) Int. Cl.: E06B 3/50, E06B 3/82, E06B 5/14, E06B 3/70, E06B 7/23

(54) **TORBLATT-EINHEIT**

(30) Priorität: 22.04.2015 DE 102015106154
(71) Anmelder: Lutz, Peter, 81925 Unterföhring (DE)
(72) Erfinder: Lutz, Peter, 81925 Unterföhring (DE)
(74) Vertreter: Weickmann & Weickmann PartmbB

(57) **Zusammenfassung**

Das Torblatt **(2)** zum Verschließen der Frontöffnung **(51)** in der Frontwand **(50**a**)** eines Trocken-Fermenters **(50)** einer Biogas-Anlage muss diese Frontöffnung **(51)** gasdicht verschließen können und zusätzlich eine hohe mechanische Stabilität aufweisen, da von Innen Biomasse mit einem ganz erheblichen Gewicht gegen das sich in der Schließstellung befindliche Torblatt anliegen kann. Statt einer bisher üblichen Stahlkonstruktion des Torblattes, das durch die korrosiven Gase im Inneren des Trockenfermenters eine kurze Lebensdauer besitzt, wird erfindungsgemäß eine Torblatt-Konstruktion vorgeschlagen, bei der der die Stabilität bietende Kern aus Holz besteht.

## Beschreibung

Die Erfindung betrifft eine Torblatt-Einheit wie sie zum dichten Verschließen eines Raumes, hier insbesondere eines Trocken-Fermenters einer Biogasanlage, benötigt wird.

Bei einem Trocken-Fermenter wird das relativ trockene, nicht fließfähige organische Material in einen Behälter eingebracht und gasdicht verschlossen, der vom Grundaufbau her meist ein hohler Kubus aus Beton ist, von dem der größte Teil der vorderen Stirnfläche, meist der kleinsten Seitenfläche des Kubus, von einer solchen Torblatt-Einheit gasdicht verschlossen werden kann, indem die Torblatt-Einheit in der Regel als eine nach oben um am Beton-Kubus angeordnete Scharniere öffnende Schwenkachsen hochgeschwenkt werden kann.

Im geschlossenen Zustand muss das Torblatt den Trocken-Fermenter gasdicht verschließen, denn das durch die Fermentierung im Inneren des Trocken-Fermenters entstehende brennbare Gas soll ausschließlich durch die dafür vorgesehenen Gas-Auslässe entnommen werden können und nicht ungenutzt in die Umgebung entweichen.

### II. Technischer Hintergrund

Zu diesem Zweck muss das Torblatt die Öffnung in der Frontwand des Fermenters einerseits gasdicht verschließen können, andererseits auch eine ausreichend große Stabilität besitzen, um selbst bei von innen dagegen drückenden organischem Material sich nicht zu verformen und dadurch die Dichtigkeit zu verlieren, was angesichts einer Größe von bis zu 10 qm und einem entsprechenden Gegendruck von innen eine erhebliche Stabilität des Torblattes erfordert.

Zu diesem Zweck sind in der Vergangenheit die Torblätter aus Stahlrahmen als tragendem Element ausgeführt gewesen, die zum Erreichen der Gasdichtigkeit auf wenigstens einer Seite, Innenseite und/oder Außenseite gasdicht, meist mit Metallblechen, beplankt wurden, die auf dem tragenden Stahlrahmen meist aufgeschweißt waren.

Dadurch lässt sich auf einfache Art und Weise eine ausreichende Stabilität erzielen, jedoch sind die sich in dem Trocken-Fermenter bildenden Gase in hohem Maße korrosiv, sodass die Lebensdauer dieser Stahlrahmen begrenzt war und bei zu starker Korrosion das gesamte Torblatt neu hergestellt und zu diesem Zweck demontiert und ein neues am Fermenter montiert werden musste, was jeweils erhebliche Ausfallzeiten nach sich zieht.

### III. Darstellung der Erfindung

### a) Technische Aufgabe

Es ist daher die Aufgabe gemäß der Erfindung, eine Torblatt-Einheit zu schaffen, die die Nachteile des Standes der Technik vermeidet und dennoch einfach und kostengünstig herzustellen und zu montieren ist.

### b) Lösung der Aufgabe

Diese Aufgabe wird durch die Merkmale des Anspruchs 1 gelöst. Vorteilhafte Ausführungsformen ergeben sich aus den Unteransprüchen.

Indem der Kern des Torblattes, der vorzugsweise zumindest zu **60**%, besser zumindest zu **70**%, besser zumindest zu **80**%, besser zumindest zu **90**%, besser alleine die Stabilität des Torblattes gewährleistet, aus Holz besteht, kann dieser Kern des Torblattes nicht durch Korrosion angegriffen und zerstört werden, da die korrosiven Gase aus dem Inneren des Fermenters das Holz nur sehr geringfügig negativ beeinflussen.

Vorzugsweise, um einen einfachen Aufbau zu gewährleisten, besteht der Kern des Torblattes deshalb aus nebeneinander verlaufenden Holzbalken, die vorzugsweise an ihren Längsseiten mit Nut und Feder ausgebildet sind und dadurch mit ihren Längsseiten formschlüssig ineinandergreifen und ein Verschieben benachbarter Holzbalken zueinander in Querrichtung zur Torblatt-Ebene vermieden wird.

Um die Dichtigkeit, insbesondere Gasdichtigkeit, des Torblattes zu gewährleisten, wird auf wenigstens einer der Hauptflächen des Kernes, insbesondere nur der Innenfläche, eine dichte Beplankung aufgebracht, die ebenfalls gasdicht ist. Diese Beplankung kann aus jedem gasdichten Material, vorzugsweise Metallblech oder Kunststoffplatten, bestehen.

In der Regel ist der Kern des Torblattes von einer um die Schmalseiten dieses Kernes umlaufenden Zarge eingefasst, die zu ihrem Inneren hin, also zum Torblatt hin, eine U-förmige Querschnittskontur aufweist, in die die schmalseitigen Randbereiche des Kernes passend formschlüssig eintauchen können.

Die Beplankung ist mit der Zarge vorzugsweise umlaufend gasdicht befestigt, insbesondere verschweisst oder verklebt, und insbesondere erstrecken sich auf der Innenseite der Beplankung angeordnete und von dieser wegweisende z.B. Gewindebolzen durch entsprechende Durchgangsöffnungen des Kernes hindurch und können auf der Gegenseite durch eine z.B. aufgeschraubte Mutter fixiert werden gegen ein Abheben der Beplankung vom Kern.

Zusätzlich kann das Profil der Zarge auf einer Seite, insbesondere der Außenseite des Torblattes, einen nach außen vorstehenden Steg aufweisen, der im geschlossenen Zustand des Torblattes an der Außenseite des die Torblatt-Öffnung umgebenden Trockenfermenters anliegt.

Vorzugsweise sind alle evtl. vorhandenen Verschraubungen am Torblatt mittels Dichtmittel oder Kleber zusätzlich gasdicht abgedichtet.

Um die Montage zu vereinfachen, besteht die umlaufende Zarge vorzugsweise aus zwei, in der Aufsicht auf die Hauptebene des Torblattes jeweils U-förmigen, Zargen-Teilen, die somit von den beiden einander gegenüber liegenden Schmal-Seiten des Torblattes aus in dessen Hauptebene gegeneinander und auf das Torblatt aufgeschoben werden können, und in diesem aufgeschobenen Zustand entweder gegeneinander und/oder gegenüber dem Kern des Torblattes verschraubt werden.

Vorzugsweise besteht in diesem Fall auch jede der Beplankungen aus zwei Platten, von denen jede den Flächenbereich eines der beiden Zargen-Teile abdecket. Auf diese Art und Weise sind die zu handhabenden Einzelteile wie Zargen-Teile und Beplankungs-Platten einfacher handhabbar als im Fall der Gesamtgröße des Torblattes.

Vorzugsweise verläuft die Berührungsebene zwischen den beiden aneinander grenzenden Platten der Beplankung und/oder zwischen den beiden aneinander grenzenden Zargen-Teilen an der Berührungsebene zwischen zwei parallel nebeneinander verlaufenden Holzbalken.

Auf diese Art und Weise kann das Torblatt in zwei Hälften vormontiert werden, jeweils bestehend aus der entsprechenden Hälfte des Kernes des Torblattes und daran bereits befestigtem Zargen-Teil und einer Platte als je eine Beplankung, sodass nach Zusammenfügen der beiden Hälften des Torblattes lediglich die aneinander grenzenden Platten und Zargen-Teile gegeneinander abgedichtet und fixiert werden müssen.

Bewegt wird das Torblatt mittels zweier parallel zueinander verlaufender Schwenkarme, die um eine gemeinsame Lagerachse mit dem Torblatt verschwenkbar sind und die nebeneinander auf der Außenseite des Torblattes angeordnet und mit dem Kern des Torblattes verbunden sind, wobei bei einem aus parallel verlaufenden Holzbalken bestehenden Kern des Torblattes die Verlaufsrichtung der Holzbalken quer, insbesondere rechtwinklig, zur Verlaufsrichtung der beiden Schwenkarme gewählt wird, insbesondere wenn die Lagerachse etwa horizontal verläuft, aber insbesondere auch, wenn die Lagerachse etwa vertikal verläuft.

Insbesondere bei einem aus zwei Hälften hergestellten Torblatt erstrecken sich die Schwenkarme über die Fuge zwischen den beiden Torblatt-Teilen hinweg und insbesondere erstrecken sich die Schwenkarme über alle den Kern bildenden Holzbalken hinweg und sind mit jedem einzelnen vorzugsweise verschraubt.

Auf einer Seite des Umfanges des Torblattes ragen die beiden Schwenkarme - die insbesondere identisch ausgebildet sind - über den Umfang des Torblattes hinaus und besitzen an diesen vorstehenden Enden - für die beiden Schwenkarme jeweils zueinander fluchtend - je zwei Lagerbuchsen, die in Verlaufsrichtung des Schwenkarmes zueinander beabstandet sind.

Mit der einen Lagerbuchse wird der Schwenkarm an einer ortsfesten Lagerstelle am Trockenfermenter schwenkbar gelagert, und an der anderen, insbesondere der vom Torblatt weiter entfernten, Lagerbuchse greift eine Schwenkeinheit an, um den Schwenkarm und damit das Torblatt um die erstgenannten Lagerbuchsen herum verschwenken zu können zwischen einer geöffneten und einer geschlossenen Stellung.

Eine solche Schwenkeinheit umfasst vorzugsweise eine Schubgabel, deren beide Zinken mit je einer der beiden Lagerbuchsen je eines der Schwenkarme gelenkig verbunden ist, während das andere Ende der Schubgabel mittels eines meist hydraulisch angetriebenen Betätigungselementes und eines Schubschlittens entlang einer Schlittenführung in einer tangentialen Richtung zur Lagerachse der ortsfest montierten Lagerbuchsen der Schwenkarme verschiebbar ist.

Durch Betätigen des z.B. wenigstens einen oder an jeder Zinke der Schubgabel vorhandenen Hydraulikzylinders und Verschieben des Schubschlittens werden die freien Enden der Zinken der Schubgabel tangential so verfahren, dass dadurch die Schwenkarme und somit das Torblatt von der offenen in eine geschlossene Stellung oder umgekehrt verschwenkt werden.

Um im geschlossenen Zustand des Torblattes ein auch am Umfang dichtes Anliegen des Torblattes am umgebenden Gebäude, also dem Trockenfermenter, zu gewährleisten, läuft üblicherweise um die Schmalseiten des Torblattes herum ein hohler, aufblasbarer Dichtungsschlauch um, der auf der Außenseite des nach innen gerichteten U-Profil-Teiles der Zarge angeordnet ist und insbesondere in eine Richtung quer zur Hauptebene an dem erwähnten Steg der Zarge anliegt, und auf der anderen Seite insbesondere ebenfalls von einem, gegebenenfalls geringer, radial nach außen vorstehenden zweiten Steg fixiert sein kann.

Zum Arretieren des Torblattes in der geschlossenen Stellung ist an wenigstens einer, vorzugsweise an zwei voneinander beabstandeten, Umfangsstellen des Torblattes auf der von der Schwenkeinheit gegenüber liegenden Seite jeweils eine zum Umfang des Torblattes hin offene Rastvertiefung vorgesehen, in die im geschlossenen Zustand ein radial von außen eingefahrener Verriegelungsbolzen, der am umgebenden Gebäude, dem Trockenfermenter, geführt und angetrieben sein kann, einfahren kann und dadurch das Torblatt in der geschlossenen Stellung verriegelt.

Diese Rastvertiefung kann durch eine auf der Außenseite des Torblattes befestigte Rasthülse zur Verfügung gestellt werden.

### c) Ausführungsbeispiele

Ausführungsformen gemäß der Erfindung sind im Folgenden beispielhaft näher beschrieben. Es zeigen:
- Fig. **1a - c:**: eine Torblatt-Einheit, montiert an einem Gebäude, in Frontansicht und Seitenansicht,
- Fig. **2**a-c:: die Toreinheit im montierten Zustand in perspektivischen Ansichten in geschlossenem und offenem Zustand sowie in einer Detaildarstellung,
- Fig. **3**a-d:: das Torblatt alleine in unterschiedlichen Ansichten,
- Fig. **4**a,b,c:: den Kern des Torblattes in unterschiedlichen Ansichten,
- Fig. **5**a:: das Torblatt in Explosionsdarstellung,

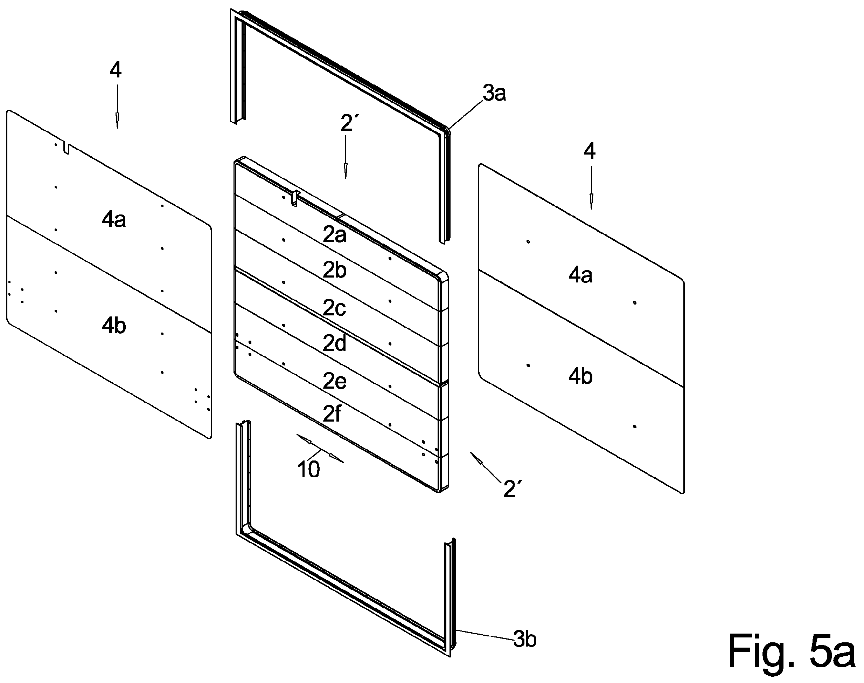

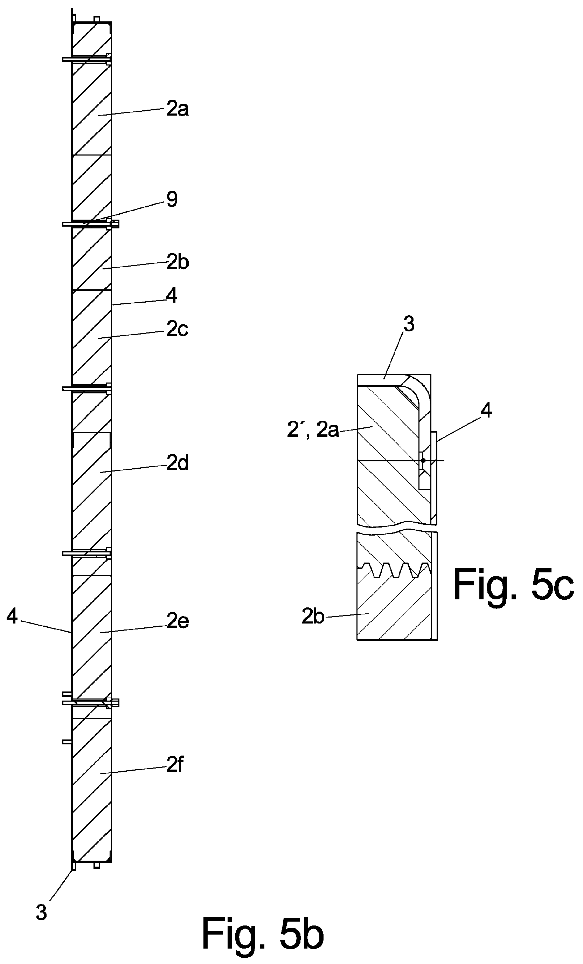

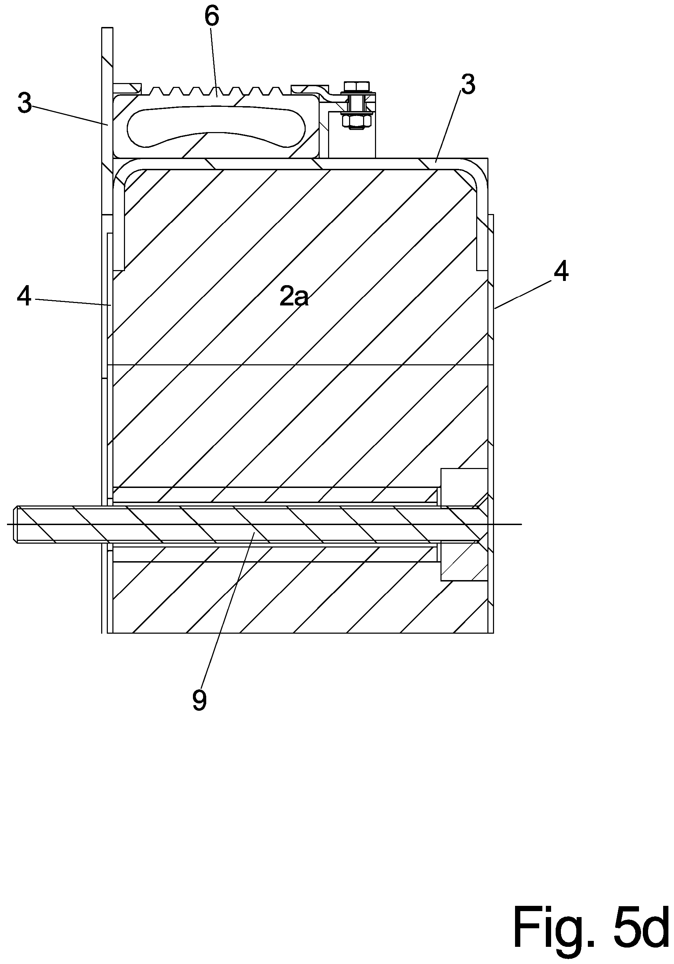

- Fig. **5**b-d:: das Torblatt in unterschiedlichen Schnittdarstellungen,

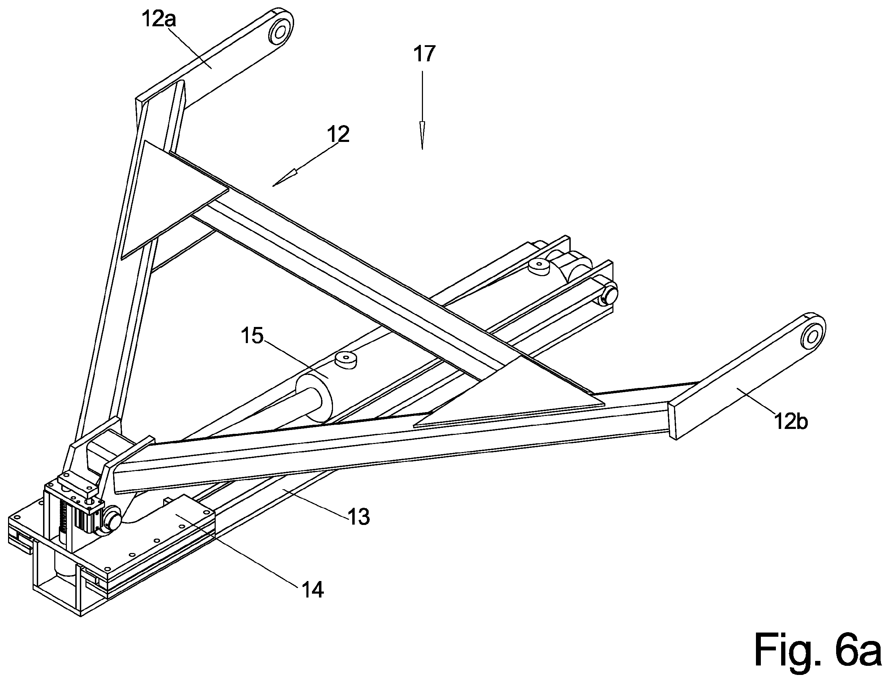

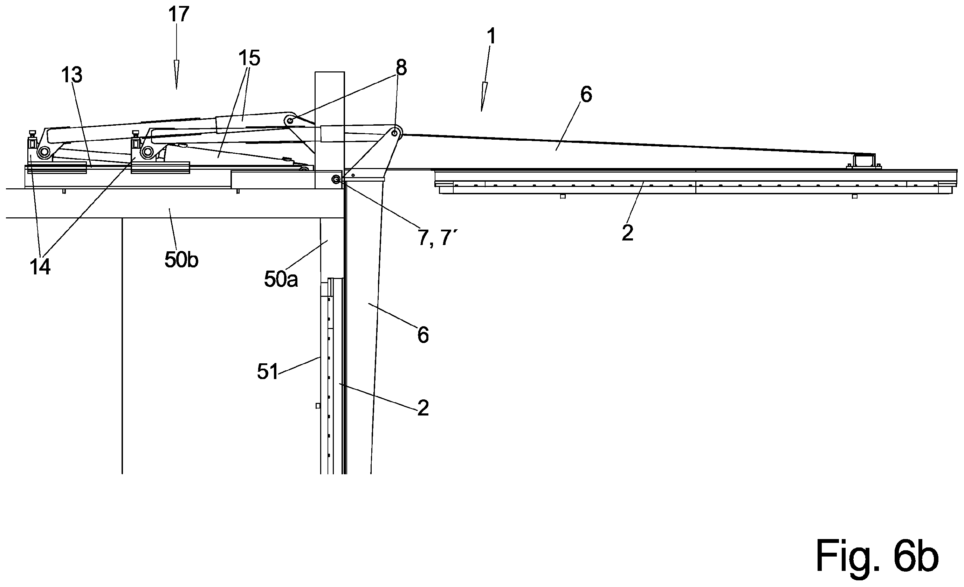

- Fig. **6a, b:**: die Schwenkeinheit für das Torblatt in unterschiedlichen Ansichten.

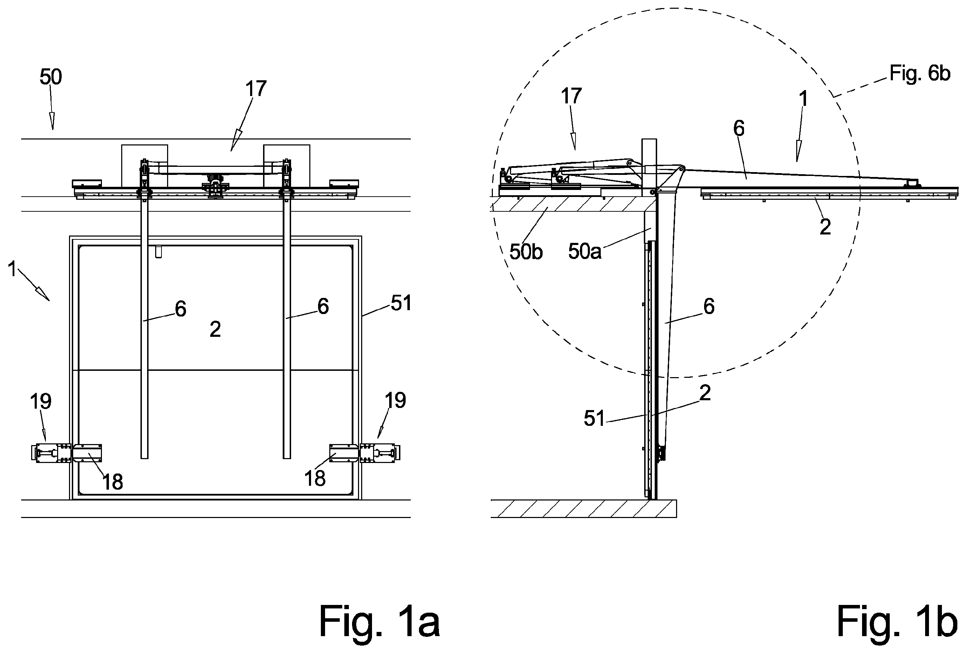

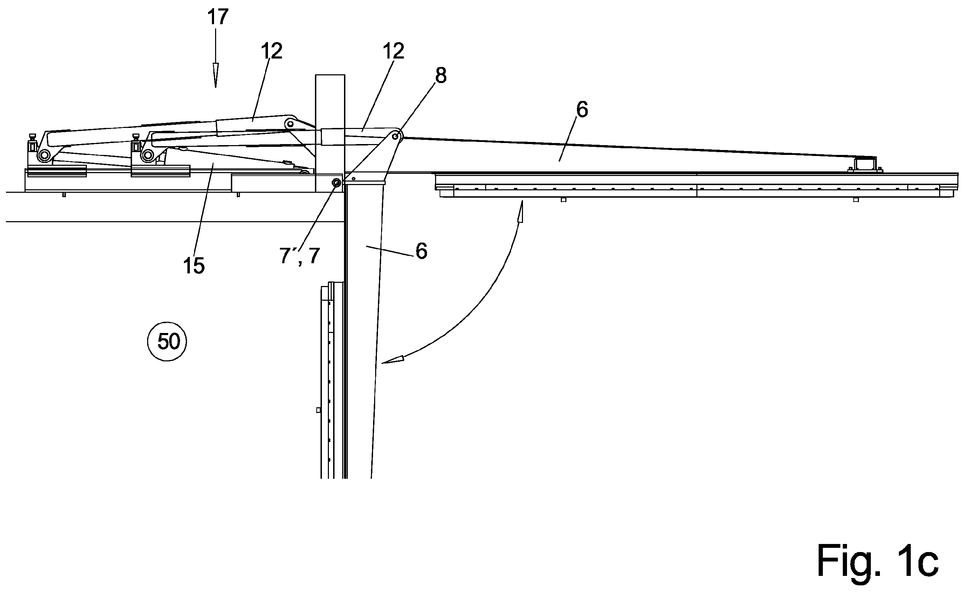

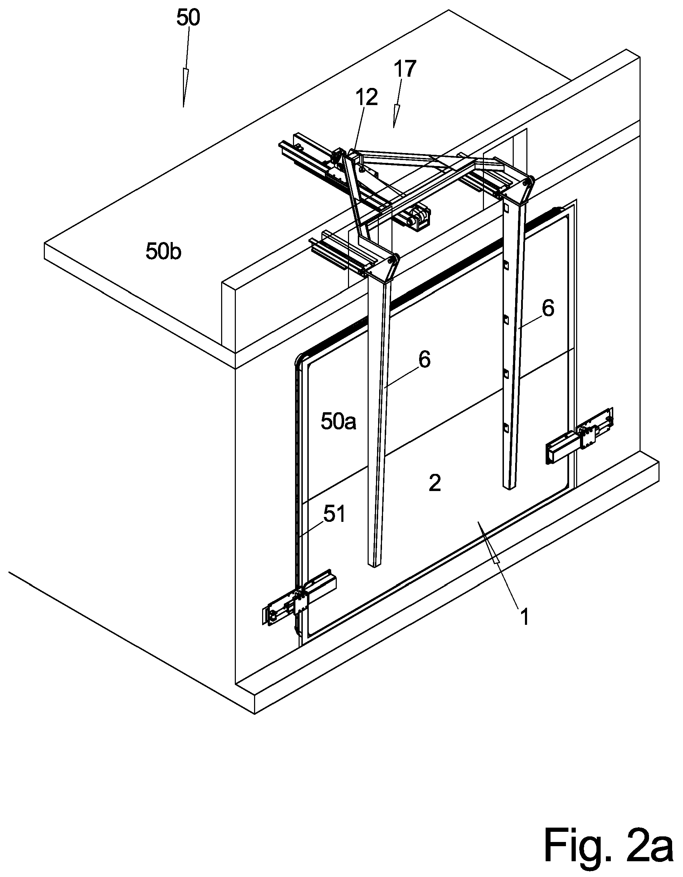

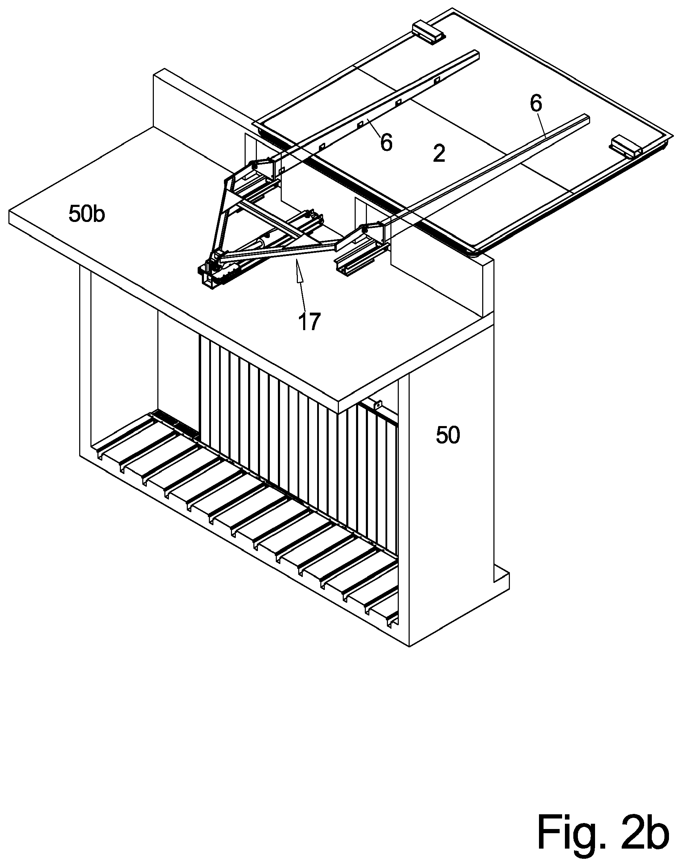

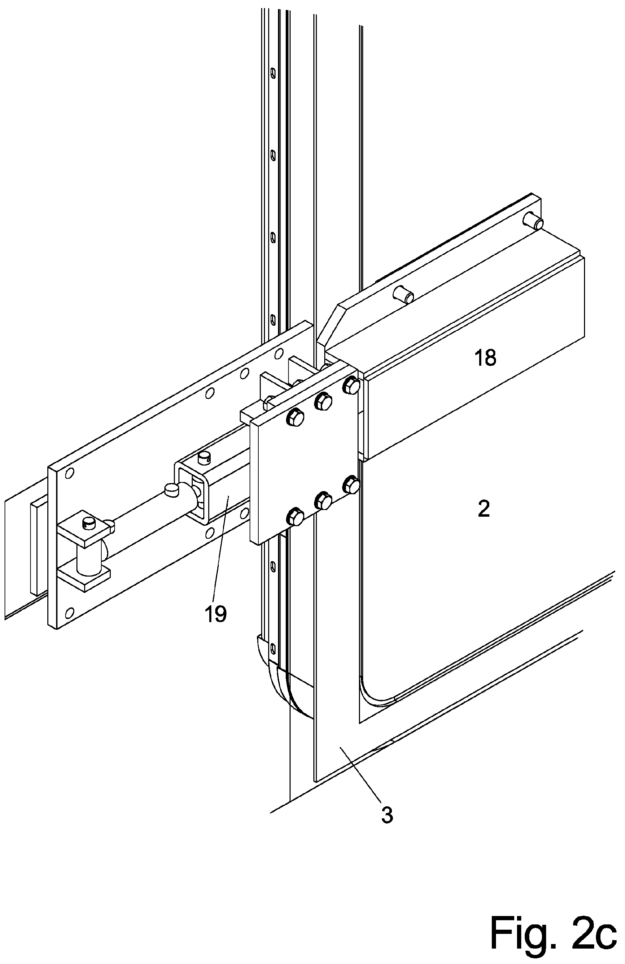

**Figur 1a** und **Figur 2a** zeigen die Torblatt-Einheit **1** montiert an einem Trockenfermenter **50** im geschlossenen Zustand, in der das Torblatt **2** die Frontöffnung **51** in der senkrecht stehenden Frontwand **50**a des Trockenfermenters dicht verschließt.

Wie **Figur 1b** zeigt, kann das Torblatt **2** der Torblatt-Einheit **1** mittels einer Schwenkeinheit **17,** die auf der Oberseite des Daches **50b** des Trockenfermenters **50** montiert ist, um eine horizontal oberhalb des Torblattes **2** liegende Lagerachse **7'** verschwenkt werden zwischen
- einer die Frontöffnung **51** verschließenden Stellung, in der der Umfang des Torblattes **2** dicht an den Innenumfangsflächen der Frontöffnung **51** in der Frontwand **50**a anliegt und
- einer geöffneten Stellung, in der das Torblatt **2** um die Lagerachse **7**' hochgeschwenkt ist in eine horizontale Lage oberhalb der Oberkante der Frontöffnung **51.**

Zu diesem Zweck sind auf der Außenseite des Torblattes **2** parallel im Abstand nebeneinander zwei Schwenkarme **6** befestigt, die in der Schließstellung des Torblattes **2** vertikal verlaufen und mit ihrem oberen Ende über die Oberkante des Torblattes **2** vorstehen und mit ihren Enden mit der Schwenkeinheit **17** verbunden sind:

Wie in einer Vergrößerung der **Figur 1b** der Schwenkeinheit **17** in **Figur 6b** besser zu erkennen, ist jeder der Schwenkarme **6** an einer Lagerbuchse **8,** die sich in der Schließstellung des Torblattes **2** oberhalb der Lagerachse **7'** befindet, mit einer der beiden Zinken **12a, b** einer etwa horizontal liegenden Schubgabel **12** verbunden, die - wie auch **Figur 6** in perspektivischer Ansicht erkennen lässt - am anderen Ende mit einem Schubschlitten **14** verbunden ist, welcher entlang einer Schlittenführung **13** horizontal verfahrbar ist, die auf der Oberseite des horizontalen Daches **50**b des Trockenfermenters **50** verschraubt ist.

Verfahren wird der Schubschlitten **14** mit einem beliebigen ansteuerbaren Betätigungselement **15,** in diesem Fall einem Arbeitszylinder **15,** etwa einem Hydraulikzylinder oder Pneumatikzylinder.

Die horizontale Lagerachse **7'** wird realisiert durch je eine weitere Lagerbuchse **7** in jedem Schwenkarm **6,** durch die die Schwenkarme **6** gemeinsam mit dem daran befestigten Torblatt **2** gegenüber dem Trockenfermenter **50** verschwenkbar sind.

Das Torblatt **2** kann in seiner Schließstellung gegenüber dem Trockenfermenter **50** verriegelt werden, indem - wie in den **Figuren 1a****,** **2a** und vor allem **2c** dargestellt - im unteren Bereich des Torblattes **2** an jeder Seitenkante eine Verriegelungseinheit vorhanden ist, die in diesem Fall in einer horizontal verlaufenden, auf der Außenseite des Torblattes **2** jeweils aufgeschraubten Rasthülse **18** besteht, die bis an oder nahe an den Seitenrand des Torblattes **2** reicht und nach außen offen ist.

An der entsprechenden Stelle ist auf der Außenseite der Frontwand **50**a des Trockenfermenters **50** ein Verriegelungsbolzen **19** in horizontaler Richtung verschiebbar so montiert, dass er zum Verriegeln vorwärts gefahren werden kann und damit in die Rasthülse **18** eintaucht oder zurückgefahren werden kann und damit aus der Rasthülse **18** und dem Schwenkbereich des Torblattes **2** herausfährt.

Der Verriegelungsbolzen **19,** kann jeweils mittels eines Betätigungselementes wie etwa eines Arbeitszylinders verschoben werden, der ebenso wie das Betätigungselement **15** für den Schubschlitten **14** von einer Steuerung aus in seiner Bewegung angesteuert werden kann.

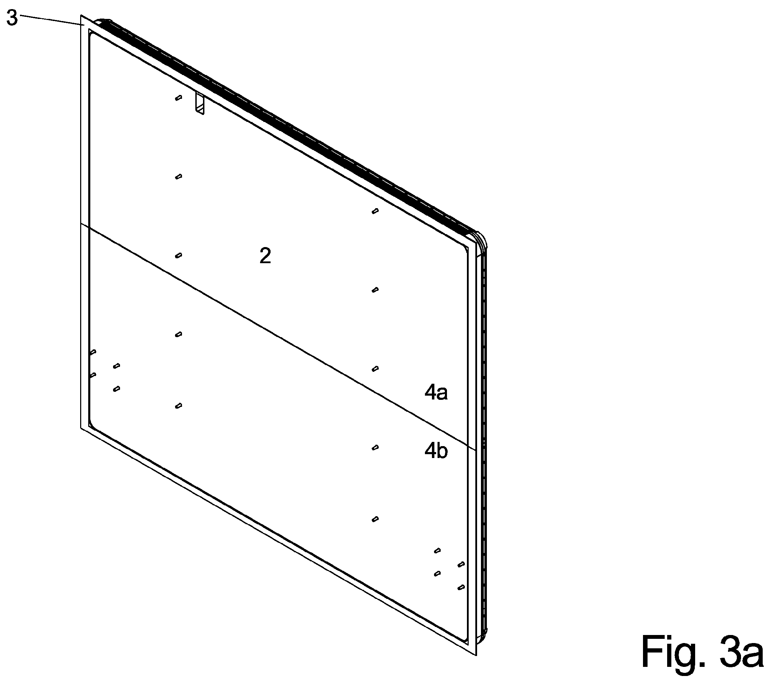

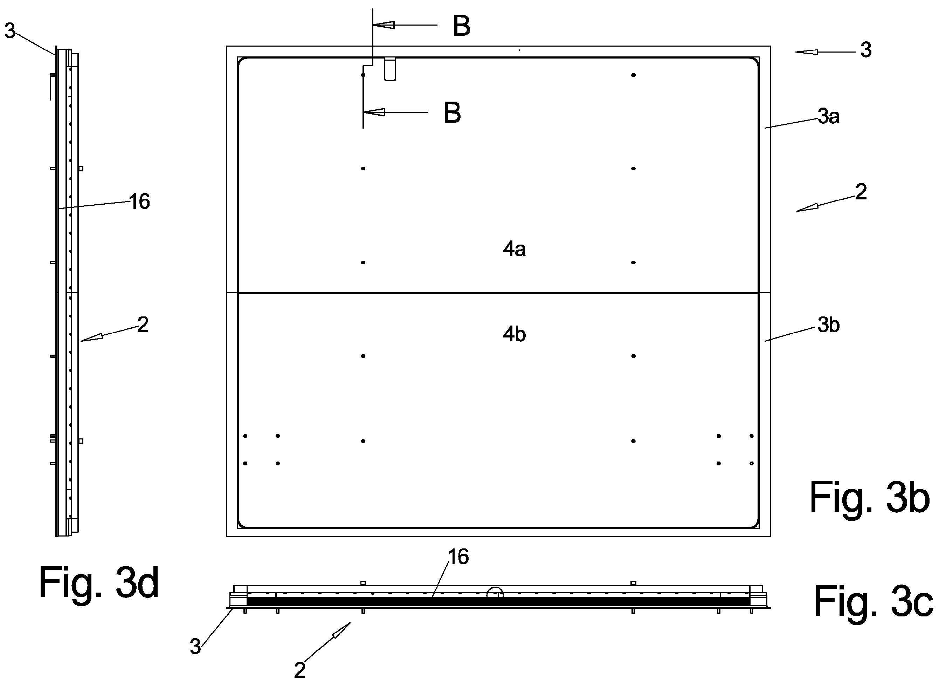

Im Mittelpunkt der vorliegenden Erfindung steht das Torblatt **2,** wie es in den **Figuren 3a****-d** in unterschiedlichen Ansichten im fertigen Zustand dargestellt ist und in Figur **5**a in Explosionsdarstellung dargestellt ist:

Um eine stabile, primär tragende, Stahlkonstruktion als Kern des Torblattes **2** zu vermeiden, weil eine solche Stahlkonstruktion anfällig für Korrosion durch die im Inneren des Biogas-Fermenters **50** vorliegenden Gasgemische ist, besteht der primär die Stabilität des Torblattes bietende Kern des Torblattes **2** aus massiven, horizontal nebeneinander verlaufenden Holzbalken **2**a, b, deren Verlaufsrichtung somit quer zur Verlaufsrichtung der später daran befestigten Schwenkarme **2** ist.

Vorzugsweise verlaufen die nebeneinander liegenden Holzbalken **2a, b...**nicht stumpf aneinander gelegt parallel nebeneinander, sondern greifen mittels einer Nut- und Federausbildung, die sich ebenfalls in Verlaufsrichtung **10** der Holzbalken erstreckt, ineinander ein, was die Gefahr minimiert, dass durch ein Verwerfung eines der Holzbalken der gesamte Kern 2' des Torblattes **2** verformt wird.

Dieser Kern **2**' ist eingefasst von einer umlaufenden Zarge **3**, die im Querschnitt eine nach innen weisende U-Kontur aufweist, in die die Schmalseiten des aus den Holzbalken **2**a, b bestehenden Kernes **2**' formschlüssig aufgenommen sind.

Durch die Zarge **3** wird eine Verwerfung der freien Enden der Holzbalken und damit der Außenseiten des Torblattes **2** unterbunden.

Die Außenseite sowie die Innenseite des Kernes **2**' ist mit einer Beplankung **4** versehen, die primär nur auf der Innenseite aus einem gasdichten Material wie Kunststoff, Edelstahl oder ähnlichem besteht und gasdicht mit der Zarge **3** verbunden ist, während die Beplankung der Außenseite primär optischen Zwecken dient und auch weggelassen werden kann.

Wie Figur **5**a erkennen lässt, ist zumindest die Beplankung **4** und die umfassende Zarge **3** jeweils in der Höhe zweigeteilt mit einer horizontal verlaufenden Trennfuge, vorzugsweise auch der Kern **2**' aus Holz:

So besteht - insbesondere jede der beiden vorderseitigen und rückseitigen - Beplankungen **4** aus jeweils einer Platte **4**a, b, die sich an einer horizontalen Kontaktebene berühren, und die Zarge **3** besteht aus zwei - betrachtet lotrecht zur Hauptebene des Torblattes **2 -** U-förmigen Hälften, von denen die eine von oben und die andere von unten auf eine Schmalseite des Kernes **2'** aufgeschoben und gegeneinander fixiert wird.

**Figur 5c** zeigt in der Schnittdarstellung, dass auf der Außenseite und auch auf der Innenseite des Kernes **2'** im Randbereich jeweils eine Ausnehmung eingearbeitet ist, in die je einer der Schenkel der nach innen gerichteten U-Form der Zarge **3** hineinpasst, sodass im montierten Zustand der Zarge **3** am Kern **2'** die Außenseite der frei endenden Schenkel des Querschnittes der Zarge **3** mit der Außenseite oder Innenseite des Kernes **2' fluchtet.**

Die Beplankung **4** erstreckt sich über den mittleren Bereich des Kernes **2'** hinweg bis auf die Zarge **3** hinaus, und ist mit dieser umlaufend gasdicht verschweißt oder verklebt.

Im mittleren Bereich ist die außenseitige und innenseitige Beplankung **4** mittels durch den Kern **2'** hindurch verlaufender Schraubbolzen **9** gegeneinander verschraubt, die aber die gasdichte innere Beplankung **4** nicht durchdringt, indem die Schraubbolzen **9** an der Rückseite der gasdichten inneren Beplankung **4** befestigt, insbesondere aufgeschweißt, sind.

Dabei zeigt **Figur 5d** im Schnitt durch den Außenbereich eines Torblattes **2,** dass die Zarge **3** nicht nur aus dem nach innen, also zur Mitte des Torblattes **2** hin, weisenden U-Profil besteht, sondern auch einen auf einer Seite, der Außenseite des Torblattes **2,** über dieses U-Profil nach außen vorstehenden Schenkel aufweist, der in der Schließstellung der Torblatteinheit **1** an den die Frontöffnung **51** umgebenden Bereichen der Außenseite der Frontwand **50**a anliegt.

Die anhand der **Figuren 1a** **und** **2a** erläuterten Schwenkarme **6** sind am Torblatt **2** ebenfalls mittels Schrauben befestigt, die sich vorzugsweise jedoch nicht durch die gasdichte innere Beplankung **4** des Torblattes **2** hindurch erstrecken.

Aus dem gleichen Grund erstrecken sich die Schwenkarme an ihren von den Lagerbuchsen **7, 8** gegenüber liegenden Enden bis in den Bereich des letzten Holzbalkens hinein und sind auch mit diesem verschraubt, insbesondere mit jedem einzelnen Holzbalken verschraubt.

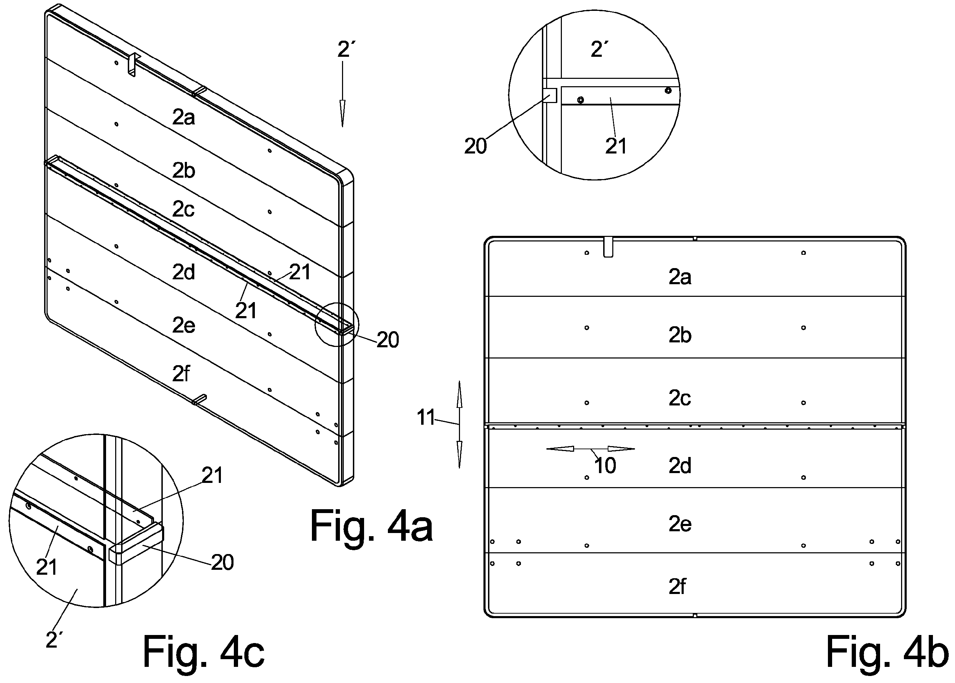

Die **Figuren 4a****-d** zeigen den aus Holz bestehenden Kern **2'** in unterschiedlichen Ansichten.

Dabei ist am besten in der Vergrößerung der Figur **4**c zu erkennen, dass zum einen in eine in horizontaler Richtung, also von der linken zur rechten Seite verlaufenden, Vertiefung sowohl in der Außenseite als auch in der Innenseite des Kernes **2'** jeweils ein langes Flacheisen **20** eingelegt und wird, welches diese Vertiefung/Nut gerade ausfüllt. Diese Nut kann sich gerade im Stoßbereich zwischen zwei Holzbalken **2**a, b, ... befinden oder auch abseits davon.

Das eingelegte lange Flacheisen **20** wird insbesondere mittels Holzschrauben gegenüber dem Kern **20'** verschraubt, und befindet sich an der Stelle der Stoßfuge der auf die jeweilige Seite, also Außenseite oder Innenseite, aufgelegten Platten **4**a, b der Beplankung, die genau in diesem Bereich gegeneinander stoßen und zur Vermeidung von Aufwölbungen nicht nur mit ihren Schmalseiten gegeneinander verschweißt werden, sondern auch mit dem darunter liegenden langen Flacheisen **20.**

Ebenso wird unter der Stoßfuge zwischen den beiden Zargen-Teilen **3**a, b über die Schmalseite des Kernes **2'** hinweg jeweils ein kurzes Flacheisen **21** in eine entsprechende Nut des Kernes **2'** eingelegt und gegenüber dem Kern **2'** verschraubt, sodass auch die genau in diesem Bereiche gegeneinander stumpf stoßenden frei endenden Schenkel der beiden Zargen-Teile **3**a, b mit dem darunter liegenden kurzen Flacheisen **21** jeweils verschweißt werden können, ohne dass beim Schweißen der aus Holz bestehende Kern **2'** zu stark angesengt wird.

Um das Torblatt **2** in der Schließstellung gegenüber der umgebenden Frontwand **50a** des Trockenfermenters **50** gasdicht abzudichten, sitzt umlaufend um das Torblatt **2** auf der Außenseite der Zarge **3**, im Winkel zu dem radial nach außen weisenden Schenkel ein hohler Dichtungsschlauch **16,** der mit Druckluft beaufschlagbar ist und durch seine Ausdehnung im Querschnitt dann dicht an der Leibung der Frontöffnung **51** anliegt sowie auch an der Außenseite des Torblattes **2.**

### BEZUGSZEICHENLISTE

- **1**: Torblatt-Einheit
- **2**: Torblatt
- **2'**: Kern
- **2**'a: oberer Teil
- **2**'b: unterer Teil
- **2**a, b: Holzbalken
- **3**: Zarge
- **3**a, b: Zargen-Teil
- **4**: Beplankung
- **4**a, b: Platte
- **5**a: Nut
- **5**b: Feder
- **6**: Schwenkarm
- **7**: Lagerbuchse
- **7'**: Lagerachse
- **8**: Lagerbuchse
- **9**: Schraube
- **10**: Verlaufsrichtung
- **11**: Querrichtung
- **12**: Schub-Gabel
- **12a, b**: Zinke
- **13**: Schlitten Führung
- **14**: Schub-Schlitten
- **15**: Betätigungselement
- **16**: Dichtungsschlauch
- **17**: Schwenk-Einheit
- **18**: Rasthülse
- **19**: Verriegelungsbolzen
- **20**: langes Flacheisen
- **21**: kurzes Flacheisen
- **50**: Trocken-Fermenter
- **50**a: Frontwand
- **50**b: Dach
- **51**: Frontöffnung

## Patentansprüche

1. Torblatt-Einheit **(1),** insbesondere für einen Trocken-Fermenter **(50)** einer Biogas-Anlage, mit einem Torblatt **(2)**
**dadurch gekennzeichnet, dass**
- der Kern (**22**) des Torblattes (**2**) aus Holz, insbesondere aus parallel nebeneinander verlaufenden Holzbalken (**2**a, b), besteht,
- der Kern (**22**) wenigstens auf der einen seiner Hauptflächen (**22**a, b) mit einer gasdichten Beplankung (**4**) versehen ist.

2. Torblatt-Einheit (**1**) nach Anspruch **1**,
**dadurch gekennzeichnet, dass**
der Kern (**22**) des Torblattes (**2**) so dimensioniert ist, dass er alleine mindestens **60**%, besser mindestens **70**%, besser mindestens **80**%, besser mindestens **90**%, besser die gesamte Stabilität des Torblattes (**2**) bietet.

3. Torblatt-Einheit (1) nach einem der vorhergehenden Ansprüche, **dadurch gekennzeichnet, dass**
die Beplankung (**4**) aus Metallblech oder Kunststoffplatten besteht und/oder
das Torblatt (**2**) um eine Lagerachse (**7**') verschwenkbar ist zwischen einer den Trocken-Fermenter (**50**) gasdicht verschließenden Schließstellung und einer den Zugang ins Innere des Trocken-Fermenters (**50**) freigebenden Offenstellung.

4. Torblatt-Einheit (**1**) nach einem der vorhergehenden Ansprüche,
**dadurch gekennzeichnet, dass**
dieser Kern (**22**) von einer entlang der Schmalseiten des Torblattes (**2**) umlaufenden Zarge (**3**) eingefasst ist, die zum Torblatt (**2**) hin eine U-förmige Querschnittskontur aufweist, die die Schmalseiten des Kernes (**22**) form schlüssig umgreift.

5. Torblatt-Einheit (**1**) nach einem der vorhergehenden Ansprüche,
**dadurch gekennzeichnet, dass**
die Beplankung (**4**) mit der Zarge (**3**) umlaufend gasdicht verbunden, insbesondere verschweißt oder verklebt, ist.

6. Torblatt-Einheit (**1**) nach einem der vorhergehenden Ansprüche,
**dadurch gekennzeichnet, dass**
die Holzbalken (**2**a, b) an ihren Längsseiten mit Nut (**5**a) und Feder (**5**b) ineinandergreifen.

7. Torblatt-Einheit (**1**) nach einem der vorhergehenden Ansprüche,
**dadurch gekennzeichnet, dass**
die umlaufende Zarge (**3**) aus mindestens zwei Zargen-Teilen (**3**a, b) besteht, die in der Aufsicht auf die Hauptebene (**2**') des Torblattes (**2**) jeweils U-förmig ausgebildet sind.

8. Torblatt-Einheit (**1**) nach einem der vorhergehenden Ansprüche,
**dadurch gekennzeichnet, dass**
die Beplankung (**4**) auf jeder Seite aus mindestens zwei Platten (**4**a, b) besteht, die jeweils den Bereich eines der beiden Zargen-Teile (**3**a, b) abdecken und insbesondere die Berührungsebene der beiden Platten (**4**a, b) mit der Berührungsebene zwischen zwei an dieser Stelle parallel nebeneinander verlaufender Holzbalken (**2**a, b) übereinstimmt.

9. Torblatt-Einheit (**1**) nach einem der vorhergehenden Ansprüche,
**dadurch gekennzeichnet, dass**
parallel zueinander beabstandet und in Querrichtung (**11**) zur Verlaufsrichtung (**10**) der Holzbalken (**2**a, b) auf der Außenseite (**22**a) des Torblattes (**2**) zwei Schwenkarme (**6**) angeordnet und mit den aus Holzbalken (**2**a, b) bestehenden Kern (**22**) des Torblattes (**2**) verschraubt sind, und die zwei Schwenkarme (**6**) auf derselben Seite über den Umfang des Torblattes (**2**) hinaus vorstehen zum Befestigen an einer Schwenkeinheit (**17**) der TorblattEinheit.

10. Torblatt-Einheit (**1**) nach einem der vorhergehenden Ansprüche,
**dadurch gekennzeichnet, dass**
die beiden Schwenkarme (**6**) identisch ausgebildet sind.

11. Torblatt-Einheit (**1**) nach einem der vorhergehenden Ansprüche,
**dadurch gekennzeichnet, dass**
an den über den Umfang des Torblattes (**2**) hinausstehenden Enden die Schwenkarme (**6**) gemessen in der Hauptebene (**2**') des Torblattes (**2**) vom Torblatt (**2**) unterschiedlich beabstandet je zwei Lagerbuchsen (**7, 8**) aufweisen, die bezüglich der beiden Schwenkarme (**6**) zueinander fluchten und von denen die jeweils eine Lagerbuchse (**7**) in einer ortsfest montierten Lagerstelle schwenkbar um eine Lagerachse (**7**') gelagert ist und die jeweils andere Lagerbuchse (**8**) gelenkig mit der Schwenk-Einheit (**17**) verbunden ist.

12. Torblatt-Einheit (1) nach einem der vorhergehenden Ansprüche,
**dadurch gekennzeichnet, dass**
die Schwenk-Einheit (**17**) eine Schub-Gabel (**12**) umfasst, die mithilfe eines entlang einer Schlittenführung (**13**) verschiebbaren Schub-Schlittens (**14**) tangential zur Lagerachse (**7**') der Lagerbuchsen (**7**) bewegbar ist, insbesondere mittels wenigstens eines, insbesondere an jeder Zinke (**12a, b**) der Schubgabel je eines, hydraulisch angetriebenen Betätigungselementes (**15**), und mit je einer der beiden Lagerbuchsen (**8**) gelenkig verbunden ist,.

13. Torblatt-Einheit (**1**) nach einem der vorhergehenden Ansprüche,
**dadurch gekennzeichnet, dass**
um die Schmalseiten des Torblattes (**2**), insbesondere der Zarge (**3**), außen umlaufend ein hohler, aufblasbarer Dichtungsschlauch (**16**) angeordnet ist, der im aufgeblasenen Zustand das Torblatt (**2**) gegen den Rest des Fermenters gasdicht abschließt.

14. Torblatt-Einheit (1) nach einem der vorhergehenden Ansprüche,
**dadurch gekennzeichnet, dass**
in dem von der Schwenk-Einheit (17) gegenüberliegenden Bereich des Torblattes (2) an wenigstens einer, insbesondere zwei einander gegenüberliegenden, Umfangstellen des Torblattes (2) eine zum Umfang des Torblattes (2) hin offene Rastvertiefung, insbesondere in Form einer Rasthülse (18) auf der Außenseite des Torblattes (2), vorgesehen ist zum Einführen eines Verriegelungsbolzens (19) und Arretieren des Torblattes (2) in der geschlossenen Stellung.
